# EUROPEAN PATENT APPLICATION

(11) **EP 3 009 515 A1**
(43) Date of publication of application: **20.04.2016**
(21) Application number: 14811598.3
(22) Date of filing: 09.06.2014
(51) Int. Cl.: C12P 7/64, C12N 1/16

(54) **PRODUCTION OF MICROBIAL OILS**

(30) Priority: 10.06.2013 ES 201330859
(71) Applicant: NEOL BIOSOLUTIONS, S.A., 18100 Armilla, Granada (ES)
(72) Inventor: CAMPOY GARCÍA, Sonia, E-18016 Granada (ES); GIBERT AMAT, Jordi, E-18016 Granada (ES); LARA CAMBIL, Armando, E-18016 Granada (ES); SUÁREZ GONZÁLEZ, Beatriz, E-18016 Granada (ES); VELASCO ALVAREZ, Javier, E-18016 Granada (ES); ADRIO FONDEVILA, José Luis, E-18016 Granada (ES)
(74) Representative: ABG Patentes, S.L.
(86) International application number: PCT/ES2014/070477
(87) International publication number: WO 2014/198988

(57) **Abstract**

The present invention relates to the *Rhodosporidium toruloides* CECT 13085 strain, as well as to uses thereof for obtaining microbial biomass rich in triglycerides and for producing oils of a microbial origin in the presence of lignocellulosic biomass hydrolysates.

## Description

### Field of the Invention

The present invention relates to the production of oils of a microbial origin from cultures of a microorganism in the presence of lignocellulosic biomass hydrolysates.

### Background of the Invention

The growing problem with CO₂ emissions in addition to concerns relating to energy safety have caused growing interest in non-petroleum based alternative energy sources in recent years. Lignocellulose is the most abundant renewable biomass source with an estimated worldwide yearly production of 10¹⁰ tons (Sánchez and Cardona, 2008. Bioresour. Technol., 99: 5270-5295), and as a result, this biomass is the only primary renewable energy source that can provide alternative biofuels such as bioethanol or biodiesel in the short term (Hamelinck et al. 2005. Biomass Bioenergy, 28: 384-410; Sánchez, 2009. Biotechnol. Avd., 27: 185-194; Zhang, 2011. Process Biochem. 46: 2091-2110; Huang et al. 2013. Biotechnol. Avd. 31: 129-139).

However, although the biological conversion of different lignocellulosic raw materials such as agricultural waste, or energy crops dedicated to the production of biofuels or chemical products offers a number of benefits, its development is still met with many technical and economic obstacles. One of the most important obstacles on both the economic and technical levels is the release of sugars present in the biomass at a low price (Lynd et al. 2008. Nature Biotechnol. 26: 169-172; Zhang, 2011. Process Biochem. 46. 2091-2110). In fact, this step is considered to be the most expensive step of the ethanol production process, and can reach up to 40% of the total process costs. Over the past few years, many pretreatment strategies for different types of raw materials have been developed, such as acid hydrolysis, alkaline hydrolysis, steam explosion, ammonia explosion or hydrothermal treatment, among others (Yang and Wyman, 2008. Biofuels Bioprod. Bior. 2: 26-40; Hendriks and Zeeman, 2009. Bioresour. Technol. 100: 10-18; Alvira et al. 2010. Bioresour. Technol. 101: 4851-4861).

One of the most important points to consider when developing a pretreatment process is to minimize the amount of toxic compounds that are generated in this step because they are potent inhibitors of microbial metabolism, affecting growth (Palmquist and Hahn-Hägerdal. 2000. Bioresour. Technol. 74: 17-24). The amount and variety of compounds depends on the type of pretreatment and the nature of the raw material due to the different degrees of methoxylation of the lignin and the association thereof with hemicellulose and cellulose. There can be three types of degradation products generated during lignocellulosic material pretreatment: carboxylic acids, furan derivatives and phenolic compounds (Palmqvist and Hagerdal. 2000. Bioresour. Technol. 74: 25-33; Almeida et al., 2007. J. Chem. Technol. Biotechnol. 82: 340-349). Acetic acid, formic acid and levulinic acid are the main weak acids. The main furan derivatives are furfural and 5-hydroxymethylfurfural, which are obtained from hexose and pentose degradation. Phenolic compounds include alcohols, aldehydes, ketones and acids (Klinke et al. 2002. Bioresour. Technol. 82: 15-26).

To minimize process costs, the product resulting from pretreatment (slurry) must be used directly to perform cellulose and hemicellulose saccharification. Furthermore, this hydrolysis process must be done using the highest possible slurry concentration (indicated as a percentage of solids). Slurry concentrations in industrial processes must at least reach values between 15-20% of solids because it allows reducing operating volumes and obtaining solutions with a high sugar concentration (120-140 g/l). However, there are two main problems when using these sugar solutions directly as part of a culture medium: firstly, the presence of the aforementioned toxic compounds (Palmquist and Hahn-Hägerdal. 2000. Bioresour. Technol. 74: 17-24; Almeida et al., 2009. Appl. Microbiol. Biotechnol. 82: 625-638); and secondly, the need to metabolize all the pentoses (fundamentally xylose) present in these mixtures for the purpose of using all the sugars and increasing the final yield of subsequent culture processes.

Although these inhibitors can be efficiently removed by means of different detoxification processes (Mussatto and Roberto. 2004. Bioresour Technol., 93: 1-10; Sánchez and Cardona. 2008. Bioresour. Technol., 99: 5270-5295; Almeida et al. 2009. Appl. Microbiol. Biotechnol. 82: 625-638), their application increases the ethanol production cost, and furthermore entails a loss of fermentable sugars. There are some hydrolysis and pretreatment processes used with raw agricultural and herbaceous materials that give rise to fermentable hydrolysates that do not require detoxification (Mosier et al., 2005. Bioresour. Technol. 96:1986-1993). However, water consumption in these cases is high, so taking into account water consumption/savings, it is necessary for the hydrolysates of any type of lignocellulosic biomass to be concentrated. As a result, it is foreseeable that the compounds formed during said process will reach inhibitory levels.

Although there are microorganisms, bacteria, filamentous fungi and yeasts capable of naturally fermenting D-xylose (Jeffries. 2006. Curr. Opin. Biotechnol. 17: 320-326; Hahn-Hagerdal et al. 2007. Appl. Microbiol. Biotechnol. 74:937-953), most microorganisms prefer glucose over other monomeric sugars (e.g., xylose) and do not assimilate the latter until glucose has been consumed (Gancedo. 1998. Microbiol. Mol. Biol. Rev. 62: 334-361). The simultaneous use of glucose and other sugars is rarely observed. However, the rapid and simultaneous use of mixtures of these sugars is considered essential for the economic viability of the production of biofuels or other chemical products (e.g., oils) from biomass hydrolysates (Rubin. 2008. Nature. 454: 841-845; Huang et al. 2013. Biotechnol. Avd. 31: 129-139). Several strategies have been used in the attempt to circumvent glucose repression, such as controlling the feed rate (Gong et al. 2012. Bioresour. Technol. 117: 20-24), reducing hexokinase activity, or by means of obtaining 2-deoxyglucose-resistant mutants (Kahar et al. 2011. J. Biosc. Bioeng. 5: 557-563).

The capacity of certain microorganisms to accumulate large amounts of lipids has been known for some years now. These microorganisms are called oleaginous microorganisms due to their similarity to the term used with the plant seeds and are defined as those capable of accumulating at least 20% of their dry weight in the form of lipids (Ratledge and Wynn. 2002. Avd. Appl. Microbiol. 51: 1-51; Ratledge. 2004. Biochemie. 86: 807-815). Among such microorganisms there are several genera of yeasts, such as *Yarrowia, Candida, Rhodotorula, Rhodosporidium, Criptococcus, Trichosporon* and *Lipomyces*. Several species belonging to these genera are capable of accumulating up to 50-70% of their dry weight in the form of lipids (Ageitos et al. 2011. Appl. Microbiol. Biotechnol. 90: 1219-1227; Beopoulos et al. 2011. Appl. Microbiol. Biotechnol. 90: 1193-1206). Lipid accumulation occurs in the presence of an excess of different carbon sources such as glucose, glycerol, whey, molasses, and even xylose in the case of species such as *Rhodosporidium toruloides* (Freer et al. 1997. Biotechnol. Lett. 19: 1119-1122; Li et al. 2010. J. Biobased Mat. Bioenergy. 4: 53-57), *Rhodotorula glutinis* (Dai et al. 2007. African J. Biotechnol. 6: 2130-2134), *Trichosporon cutaneum* (Chen et al. 2009. Appl. Biochem. Biotechnol. 159: 276-290) or *Trichosporon fermentans* (Huang et al. Bioresour. Technol. 100: 4535-4538), among others.

*Rhodosporidium toruloides* is one of the oleaginous organisms with the greatest potential for the development of industrial processes due to its capacity to grow to high cell densities, easy scaling and its capacity to accumulate lipids, mostly in the form of triglycerides (WO2009118438; Zhao et al. 2010. J. Ind. Microbiol. Biotechnol. 1: 1-6; Ageitos et al. 2011. Appl. Microbiol. Biotechnol. 90: 1219-1227).

However, *Rhodosporidium toruloides* is not capable of growing directly in sugarcane bagasse or wheat straw hydrolysates, prepared from 10% solids (Yu et al. 2011. Bioresour. Technol. 102: 6134-6140; Zhao et al. 2012. Bioprocess Biosyst. Eng., 35: 993-1004), so processes removing these toxic compounds must be performed in order to produce lipids. The viability of this yeast is affected, to a different extent, by some of these inhibitory compounds, particularly by furfural and its derivatives, furfuryl alcohol and furoic acid (Hu et al. 2009, Bioresour. Technol., 100: 4843-4847).

Taking all this into account, in an industrial context, obtaining a robust microorganism that is resistant to the action of these inhibitory compounds, capable of rapidly metabolizing xylose and accumulating a large amount of lipids, is an indispensable prerequisite for being able to approach the development of a microbial lipid production process based on very inexpensive sugar sources, such as lignocellulosic biomass hydrolysates.

### Brief Description of the Invention

The authors of the present invention have isolated a microorganism of the *Rhodosporidium toruloides* CECT 13085 strain which has the capacity to efficiently metabolize different carbon sources including, without limitation, glucose, xylose, crude glycerine or sugars present in lignocellulosic biomass hydrolysates without detoxification, and to furthermore accumulate lipids up to at least 50% of the dry weight.

Therefore, in a first aspect the present invention relates to a microorganism of the *Rhodosporidium toruloides* CECT 13085 strain or of a mutant strain thereof, which maintains the capacity to accumulate lipids up to at least 50% of the dry weight, the capacity to be resistant to biomass hydrolysates without detoxification and the capacity to metabolize xylose.

In another aspect, the present invention relates to a method for obtaining a microbial biomass rich in triglycerides, comprising
i) culturing a microorganism according to the first aspect of the invention in a culture medium comprising at least one carbon source and at least one nitrogen source in conditions suitable for growth of said microorganism, and
ii) separating the microbial biomass from the culture medium.

In another aspect, the present invention relates to a method for extracting lipids from the microbial biomass according to the preceding aspect, comprising a mechanical extraction method or a solid-liquid extraction method.

In another aspect, the present invention relates to a method for obtaining paraffins, comprising
i) obtaining a lipid-enriched preparation from the microbial biomass of the invention
ii) refining said lipids,
iii) converting the mixture of refined lipids obtained in step ii) into paraffins.

In another aspect, the present invention relates to a method for obtaining biodiesel, comprising
i) obtaining a lipid-enriched preparation from the microbial biomass of the invention
ii) refining said lipids,
iii) converting the mixture of refined lipids obtained in step ii) into biodiesel.

In another aspect, the invention relates to a method for obtaining biolubricants, comprising:
i) obtaining a lipid-enriched preparation from the microbial biomass of the invention
ii) refining said lipids,
iii) converting the mixture of refined lipids obtained in step ii) into biolubricants.

In another aspect, the invention relates to a method for obtaining biosurfactants, comprising:
i) obtaining a lipid-enriched preparation from the microbial biomass of the invention
ii) refining said lipids,
iii) converting the mixture of refined lipids obtained in step ii) into biosurfactants.

In another aspect, the present invention relates to the use of the microorganism according to the invention for obtaining a microbial biomass rich in triglycerides according to the first method of the invention.

In another aspect, the present invention relates to the use of the microorganism according to the invention for extracting lipids from the microbial biomass according to the second method of the invention.

In another aspect, the present invention relates to the use of the microorganism according to the invention for obtaining paraffins according to the third method of the invention.

In another aspect, the present invention relates to the use of the microorganism according to the invention for obtaining biodiesel according to the third method of the invention.

In another aspect, the present invention relates to the use of the microorganism according to the invention for obtaining biolubricants according to the fourth method of the invention.

In another aspect, the present invention relates to the use of the microorganism according to the invention for obtaining biosurfactants according to the fifth method of the invention.

### Breve Description of the Drawings

Figure 1 shows the resistance curve with respect to the inhibitory compounds of the *R. toruloides* 0013-09 (indicated in black) and 0041-12 (indicated in red) strains cultured in HB medium (m/MBO_008_1 1 medium containing wheat straw hydrolysate, 20% of solids) (solid lines) and in m/MBO_007 medium (without hydrolysate) (discontinuous lines).
Figure 2 shows consumption curves with respect to glucose (circles) and xylose (squares) sugars of the *R. toruloides* 0041-12 (upper panel), M133 (middle panel) and M709 (lower panel) strains.

### Detailed Description of the Invention

### Microorganism of the invention

In a first aspect, the present invention relates to a microorganism, hereinafter "microorganism of the invention", in which the microorganism belongs to the *Rhodosporidium toruloides* CECT 13085 strain or to a mutant strain thereof, which maintains the capacity to accumulate lipids up to at least 50% of the dry weight, the capacity to be resistant to biomass hydrolysates without detoxification and the capacity to metabolize xylose.

As it is used herein, the term "microorganism" or "microbe" refers to a single-celled or multicellular microscopic organism with the capacity to accumulate lipids intracellularly. Particularly, the microorganism of the invention is a yeast of the *Rhodosporidium toruloides* species, specifically the CECT 13085 strain.

As it is used herein, the term "strain" refers to a genetic variant or subtype of a certain organism.

As it is used herein, the term "mutant strain" refers to a strain resulting from mutation of a strain of a certain organism and substantially maintaining the properties of said strain.

The microorganism of the invention refers to a mutant of the *Rhodosporidium toruloides* CECT 13085 strain substantially maintaining the capacity of the *Rhodosporidium toruloides* CECT 13085 strain to accumulate lipids up to at least 50% of the dry weight, the capacity to grow in the presence of biomass hydrolysates without detoxification and the capacity to metabolize xylose.

The microorganism of the invention also refers to a mutant strain thereof which maintains the capacity to accumulate lipids up to at least 50% of the dry weight. In a particular embodiment, the mutant strain of the *R. toruloides* CECT 13085 strain maintains the capacity to accumulate lipids up to at least 50% of the dry weight, at least 60% of the dry weight, at least 70% of the dry weight, at least 80% of dry weight or at least 90% of the dry weight.

As known by the person skilled in the art, the capacity to accumulate lipids can be analyzed by means of many methods available in the art. These methods include, without limitation, determining the total lipid content by means of methods of extraction with organic solvents (for example Soxhlet, Goldfish, Mojonnier), or total lipid content can also be quantified by methods of extraction that do not include solvents (for example Babcock, Gerber) and by instrumental methods based on physical or chemical lipid properties (for example infrared, density and X-ray absorption).

The microorganism of the invention also refers to a mutant strain of the CECT 13085 strain which maintains the capacity to grow in the presence of biomass hydrolysates without detoxification.

As it is used herein, the term "biomass hydrolysate" refers to any saccharification product containing the sugars produced in the saccharification process, non-hydrolyzed biomass residues and enzymes used for said biomass hydrolysis.

As it is used herein, the term "saccharification" or "biomass hydrolysis" refers to the production of fermentable sugars from polysaccharides.

As it is used herein, the term "fermentable sugar" refers to oligosaccharides and monosaccharides that can be used as a carbon source by a microorganism in the fermentation process to obtain products such as ethanol.

As they are used herein, the terms "biomass" and "biomass substrate" refer to any material suitable for use in saccharification reactions. Said terms include but are not limited to materials comprising cellulose (for example cellulosic biomass, cellulosic raw material and cellulosic substrate), lignin or the combination of cellulose and lignin. The biomass can be derived from plants, animals or microorganisms and can include, without being limited to, agricultural, industrial and forestry waste, agricultural and municipal waste products and energy crops resulting from land farming and aquafarming. Examples of biomass substrates include but are not limited to wood, wood pulp, paper pulp, corn fiber, corn grain, corncobs, crop waste such as corn husks, corn stubble, fodder, wheat, wheat straw, barley, barley straw, hay, rice, rice straw, millet, paper waste, paper, waste from processing pulp, woody plants or herbaceous plants, fruit or vegetable pulp, products resulting from the distillation of grain, grasses, rice hulls, cotton, hemp, linen, sisal, sugarcane bagasse, sorghum, soybean, millet, components obtained from grinding grains, trees, branches, roots, leaves, wood shavings, sawdust, bushes and shrubs, vegetables, fruits and flowers, and any combination thereof. In some embodiments, the biomass comprises, but is not limited to, cultivated plants (for example grasses, including C4 grasses, such as switchgrass, cordgrass, ryegrass, *Miscanthus,* reed canary grass or combinations thereof), waste from processing sugar, for example, but without being limited to, bagasse [for example, sugarcane bagasse, beet (for example sugar beet) pulp, or a combination thereof], agricultural waste (for example soybean stubble, corn stubble, corn fiber, rice straw, sugarcane straw, rice, rice hulls, barley straw, corncobs, wheat straw, canola straw, oat straw, oat hulls, corn fiber, hemp, linen, sisal, cotton or any combination thereof), fruit pulp, vegetable pulp, products resulting from distillation of grain, forest biomass (for example wood, wood pulp, fiber, recycled wood pulp fibers, sawdust, hard wood, such as poplar wood, soft wood, or a combination thereof).

In some embodiments, the biomass comprises of cellulosic waste product material and/or forestry waste material including, but without being limited to, paper and waste from processing paper pulp, municipal paper waste, newspaper paper, cardboard and the like. In some embodiments, the biomass comprises one species of fiber, whereas in other alternative embodiments the biomass comprises a mixture of fibers originating from different biomasses. In some embodiments, the biomass can also comprise transgenic plants expressing ligninase and/or cellulases (see patent document US2008/0104724 A1, for example).

The term "biomass" includes any living or dead biological material containing polysaccharides as substrates, including, but without being limited to, cellulose, starch, other forms of long-chain carbohydrate polymers and combinations thereof. The biomass may or may not be formed entirely from glucose or xylose, and it can optionally contain other pentose or hexose monomers. Xylose is an aldopentose containing five carbon atoms and an aldehyde group. It is the hemicellulose sugar precursor and is often the main biomass component. In some embodiments, the substrate is placed in a suspension before pretreatment. In some embodiments, the consistency of the suspension is between about 2% and about 30%, and more typically between about 4% and about 15%. In some embodiments, the suspension is washed or treated with acid before pretreatment. In some embodiments, the suspension is dehydrated by means of any suitable method for reducing water and chemical product consumption before pretreatment. Examples of dehydration devices include, but are not limited to, pressurized screw presses (see patent document WO 2010/022511, for example), pressurized filters and extruding presses.

A biomass substrate is "pretreated" when it has been subjected to physical and/or chemical methods to make saccharification easier. In some embodiments, the biomass substrate is "pretreated" or "treated" to increase the susceptibility of said biomass to cellulose hydrolysis by means of using methods known in the state of the art (Cuervo et al., Biotecnologia, 2008, 13:3), such as physicochemical pretreatment methods (for example treatment with ammonium, pretreatment with diluted acid, pretreatment with diluted alkalis, exposure to solvents, steam explosion, grinding, extrusion), biological pretreatment methods (for example applying lignin-solubilizing microorganisms) and combinations thereof.

Grinding consists of a process of milling plant material until it is reduced to particles of different sizes that can be separated by mechanical methods.

Extrusion is a method whereby plant material is forced to flow, under one or more of a variety of mixing, heating and shearing conditions, through a nozzle designed to give shape to or expand the ingredients. It can be done in cold conditions, where the material is extruded without expansion, or in hot conditions, in which the macromolecules of the components lose their discontinuous native structure and in which a continuous viscous mass that dextrinizes and gelatinizes starch is formed, proteins are denatured, enzymes responsible for possible deteriorations are inactivated, some non-nutritional compounds are destroyed and the microbial load is destroyed.

Acid hydrolysis consists of treating plant material with acids such as sulfuric acid or hydrochloric acid using high temperatures. Cellulose hydrolysis is favored by means of this process, but it requires neutralizing the pH when hydrolysis ends to allow subsequent growth of microorganisms.

Treatment with alkalis consists of adding diluted bases to the plant biomass. Efficiency of this method depends on the lignin content in the materials. Diluted sodium hydroxide causes swelling, which allows an increase in the internal surface area, reducing the degree of cellulose polymerization and crystallinity, causing the structural attachments between lignin and carbohydrates to separate.

Treatment with organic solvents consists of using solvents such as methanol, ethanol or acetone to break up lignin and cellulose bonds. It is necessary to remove the solvents from the system because they inhibit growth of the organisms.

Treatment with ionic liquids (for example with a sodium chloride solution) favors cellulose degradation because the hydrogen and oxygen atoms forming part of same separately interact with the solvent such that hydrogen bridge bonds between cellulose chains are broken up.

Treatment with steam explosion consists of treating the biomass with saturated steam at a temperature of 160-260°C (0.69-4.83 MPa) for a certain time which will depend on the type of source plant material.

Treatment with lignin-solubilizing microorganisms consists of treating the biomass with microorganisms producing enzymes with the capacity to degrade lignocellulosic material, such as, for example, *Trichoderma reesei, Fusarium oxysporium, Piptopus betulinus, Penicillum echinalatum, Penicillum purpurogenum*, *Aspergillus niger, Aspergillus fumigatus, Anaeromyces sp*., *Caecomices sp., Cyllamcyces sp., Neocallimastix sp., Orpinomyces sp*., *Piromyces sp*., *Sporotrichum thermophile, Scytalidium thermophillum, Thermonospora cubata, Rhodosporillum rubrum, Cellulomonas fimi, Clostridium stercocarium, Bacillus polymyxa, Pyrococcus furiosus, Acidothermus cellulotycus, Saccharophagus degradans,* etc.

As it is used herein, the term "biomass hydrolysate without detoxification" refers to the hydrolysate containing all the degradation products which are generated during the biomass hydrolysis process and which are generally carboxylic acid type products (particularly acetic acid, formic acid and levulinic acid), furan derivatives (particularly furfural and 5-hydroxymethylfurfural) and phenolic compounds (particularly alcohols, aldehydes, ketones and acids, and more particularly coumaric acid, succinic acid, 4-hydroxybenzoic acid, catechol, guaicol, syringic acid, ferulic acid, 4-hydroxybenzaldehyde, vanillin, vanillic acid, syringaldehyde) and combinations thereof.

As it is used herein, the term "capacity to grow in biomass hydrolysate without detoxification" refers to the fact that the strain is capable of growing in a medium containing biomass hydrolysate without detoxification up to concentrations greater than those of the *R. toruloides* 0041-12 parental strain in the same conditions. In a preferred embodiment, the strain according to the invention is capable of growing in small-scale cultures up to an optical density measured at 600 nm of at least 50 and/or a biomass density of 16 g/l of biomass. Mutant strains of the invention are therefore capable of growing up to cell concentrations exceeding cell concentrations that would be obtained in the same medium and under the same conditions starting from the 0041-12 parental strain by at least 10%, at least 20%, at least 30%, at least 40%, at least 50%, at least 60%, at least 80%, at least 90%, at least 100% or more. In a preferred embodiment, the culture medium in which growth capacity is determined contains at least 10%, at least 20%, at least 30%, at least 40%, at least 50%, at least 60%, at least 70% or more of biomass hydrolysate without detoxification.

Suitable methods for determining the capacity of a strain to grow in the presence of biomass hydrolysates without detoxification include, for example, the method described in Example 2 of the present invention based on the capacity of the strain to grow in m/MBO_008_1 culture medium comprising 9.6 g/l corn steep liquid and 20-100 g/l sugars, pH 6.

The microorganism of the invention refers to a mutant of the *Rhodosporidium toruloides* CECT 13085 strain substantially maintaining the capacity to metabolize xylose. As it is used herein, the expression "capacity to metabolize xylose" refers to the capacity of the microorganisms to grow in the presence of xylose as the only carbon source. In a preferred embodiment, the strain is capable of growing in the presence of 20 g/l of xylose. In another preferred embodiment, the strain is capable of growing in the presence of 40 g/l of xylose. In a preferred embodiment, the microorganism according to the present invention is capable of growing at least 10%, at least 20%, at least 30%, at least 40%, at least 50%, at least 60%, at least 70%, at least 80, at least 90% or more with respect to the *Rhodosporidium toruloides* strain from which it is derived.

Suitable methods for determining the capacity of a microorganism to metabolize xylose include, for example, the method described in Example 3 of the present invention, based on the capacity of the strain to grow in culture media in which xylose is the only available carbon source. In a preferred embodiment, the strain according to the invention is capable of growing in a culture medium containing xylose as the only carbon source at a concentration of at least 20 g/l or at least 40 g/l.

The *R. toruloides* CECT 13085 strain has the capacity to metabolize various carbon sources other than xylose, including, without limitation, glucose, crude glycerine or sugars present in lignocellulosic biomass hydrolysates.

As it is used herein, the term "lignocellulosic biomass" refers to a composition comprising both lignin and cellulose. In some embodiments, the lignocellulosic material can also comprise starch. "Lignin" is a polyphenolic material. Lignins can be highly branched and can also be cross-linked. Lignins can have a significant structural variation which depends, at least in part, on the source of the plant at hand. Lignocellulosic materials include a variety of plants and plant materials, such as, without limitation, paper manufacture sludge, wood, and materials relating to wood, for example, sawdust or particle boards, leaves or trees such as poplars, grasses such as whole-grain millet; planting corn, sorghum, Sudan grass, grass clippings, rice hull, bagasse (for example sugarcane bagasse), jute, hemp, linen, bamboo, sisal, abaca, hay, straw, corncobs, corn and sorghum stubble, and the coir.

### Method for obtaining a microbial biomass rich in triglycerides

In another aspect, the present invention relates to a method for obtaining a microbial biomass rich in triglycerides, hereinafter "first method of the invention", comprising
i) culturing the microorganism of the invention in a culture medium comprising at least one carbon source and at least one nitrogen source in conditions suitable for growth of said microorganism, and
ii) separating the microbial biomass from the culture medium.

As it is used herein, the term "microbial biomass" refers to the biological material derived from living or recently living organisms, particularly from the microorganism of the invention, and to the organic material proceeding from a spontaneous or provoked biological process that can be used as an energy source. As a renewable energy source, biomass can be used direct or indirectly, after being converted into another type of product such as biofuel. In the particular case of the present invention, the microbial biomass is rich in triglycerides. As it is used herein, the term "microbial biomass rich in triglycerides" refers to a microbial biomass with a content of at least 50%, at least 60%, at least 70% or at least 80% of its total dry weight.

In a first step, the first method of the invention comprises culturing the microorganism of the invention in a culture medium comprising at least one carbon source and at least one nitrogen source in conditions suitable for growth of said microorganism.

As it is used herein, the term "culturing" refers to the method of seeding, maintaining and making microorganisms develop on suitable culture media.

As it is used herein, the term "culture medium" refers to a liquid, semisolid or solid medium having the nutrients necessary for allowing growth of microorganisms in favorable pH, temperature and oxygenation conditions. In a particular embodiment, the culture medium is a liquid medium. Culture media suitable for culturing microorganisms are well known in the art, such as, for example, Maniatis et al. (1982, Molecular Cloning, A Laboratory Manual, Cold Spring Harbor Laboratory, NY) and Madigan & Martinko (2005, Brock Biology of Microorganisms, 11th ed.). Among other nutrients, the culture medium comprises a carbon source and a nitrogen source. Non-limiting examples of culture media suitable for carrying out the first method of the invention include MEM medium; YPD medium; m/MBO_007 medium (60 g/l glucose, 40 g/l xylose, 9.6 g/l corn steep liquid, pH 6); m/MBO_008 medium (lignocellulosic biomass hydrolysate up to a sugar concentration of 20 g/l, 9.6 g/l corn steep liquid, pH 6); m/MBO_008_1 medium (composition: 9.6 g/l corn steep liquid, 70 g sugars/l of wheat straw hydrolysate, pH 6); m/MBO_008_2 medium (composition: 9.6 g/l corn steep liquid, 70 g sugars/l of sugarcane bagasse hydrolysate, pH 6); or m/MBO_008_3 medium (composition: 9.6 g/l corn steep liquid, 70 g sugars/l of hydrolysate of oil palm empty fruit bunches, pH 6); m/MBO2_003 medium (10 g/l glucose, 10 g/l xylose, 9.6 g/l corn steep liquid, pH 6); m/MBO_002 medium (in g/l: 1 NH₄NO₃, 0.4 CaCl₂•H₂O, 0.76 KH₂PO₄, 0.4 MgSO₄•7H₂O, 20 xylose, 20 agar, pH 6)

In a particular embodiment, the carbon source is a lignocellulosic biomass hydrolysate. The terms "biomass hydrolysate", "biomass" and "lignocellulosic" have already been defined in the context of the microorganism of the invention.

As the skilled person will understand, the lignocellulosic biomass hydrolysate can be obtained from different plant sources or byproducts thereof. As it is used herein, the term "byproduct" refers to the product resulting from subjecting said plant to physical and/or chemical methods. In a particular embodiment, the culture medium comprises as a carbon source a lignocellulosic biomass hydrolysate which is obtained from wheat straw, sugarcane bagasse, oil palm empty fruit bunches, oil palm pruning, oil palm fiber, grapevine pruning, olive tree pruning and combinations thereof. In a preferred embodiment, said hydrolysate comes from wheat straw. In another preferred embodiment, said hydrolysate comes from sugarcane bagasse. In another preferred embodiment, said hydrolysate comes from oil palm empty fruit bunches.

Said aforementioned combinations of hydrolysates preferably have at least 5%, at least 10%, at least 20%, at least 30%, at least 40% of hydrolyzed lignocellulosic biomass.

In another particular embodiment, the carbon source used for culturing the microorganism of the invention comes from a mixture of a lignocellulosic biomass hydrolysate and glycerol. As the skilled person will understand, the proportion of hydrolysate and glycerol may vary so that the lipid production and growth conditions of the microorganism of the invention are optimal. Therefore, the biomass hydrolysate:glycerine ratio is preferably 60:40; the biomass hydrolysate:glycerine ratio is more preferably 70:30; and the biomass hydrolysate:glycerine ratio is even more preferably 75:25.

In another particular embodiment, the carbon source is selected from the group consisting of glucose, glycerol, glycerine, molasses, xylose, arabinose, mannose, fructose, acetate, starches and combinations thereof. In a preferred embodiment, the carbon source is glucose. In another preferred embodiment, the carbon source is xylose. In a more preferred embodiment, the xylose concentration in the culture medium is 20 g/l.In another more preferred embodiment, the xylose concentration in the medium is 40 g/l.

In another particular embodiment, the source of the nitrogen source is selected from the group consisting of yeast extract, peptone, corn steep liquid, urea, sodium glutamate, different inorganic nitrogen sources, such as ammonium salts and combinations thereof. In a preferred embodiment, the nitrogen source is an ammonium salt, preferably ammonium chloride.

In another particular embodiment, the culture medium comprises solid inhibitors. As it is used herein, the term "solid inhibitors" refers to compounds inhibiting the microbial metabolism and negatively affecting growth of the organism. In a more particular embodiment, said solid inhibitors come from degradation of the biomass without detoxification (for example, they come from lignocellulose degradation) and are selected from the group consisting of acetic acid, formic acid, levulinic acid, coumaric acid, ferulic acid, succinic acid, 4-hydroxybenzaldehyde, vanillin, vanillic acid, syringaldehyde, 4-hydroxybenzoic acid, catechol, guaicol, syringic acid, furfural, 5-hydroxymethylfurfural and combinations thereof.

Suitable methods for determining the capacity of an R. *toruloides* strain to grow in the presence of solid inhibitors coming from biomass hydrolysates without detoxification include, for example, methods that allow determining the adaptation of the microorganism to a culture medium in which the inhibitor concentration is progressively increased, such as the method shown in Example 2 of the present invention.

The microorganism culture can normally be subjected to metabolic stress, such that the microorganisms intracellularly produce and accumulate large amounts of lipids. Metabolic stress can be induced by an excess carbon source in relation to the nitrogen source in the culture medium. Triglyceride accumulation takes place when a there is an excess carbon source and the nitrogen source limits growth. Under these growth conditions, cells use the carbon source for neutral lipid synthesis.

The methods for culturing microorganisms are standard in the art and are well known by the skilled person. The culture can be carried out in flasks or bioreactors until reaching a high triglyceride content, typically equal to or greater than 50% by dry weight. The duration of the culture varies, although culture typically lasts from 3 to 7 days.

As it is used herein, the term "conditions suitable for growth of the microorganism of the invention" refers to conditions that support growth of the microorganism of the invention. Such conditions can include pH, nutrients, temperature, moisture, oxygenation, environment and other factors.

In a particular embodiment, the conditions suitable for growth of said microorganism of step i) comprise
- a temperature in a range between 18°C and 37°C, preferably between 23°C and 32°C, more preferably between 28°C and 30°C,
- a dissolved oxygen concentration of at least 20%, and/or
- constant stirring.

The conditions in which the microorganism of the invention is cultured can be adjusted to increase the percentage of triglycerides per unit of dry weight in the resulting microbial biomass. For example, it is possible to culture the microorganism in the presence of limiting concentrations of a nutrient, such as nitrogen, phosphorus or sulfur, for example, while at the same time maintaining an excess carbon source. The limitation of the nitrogen source allows increasing the lipid yield of the biomass per unit of dry weight. The microorganism can be cultured in limiting conditions that limit any of the nutrients throughout the entire culture time, or it can be cultured by alternating culture cycles at limiting concentrations and culture cycles without limiting concentrations.

The culture according to the first method of the invention is performed until the desired amount of biomass has been achieved and/or until the biomass contains the desired intracellular amount of triglycerides. The skilled person will understand that it is possible to monitor the culture to determine the amount of biomass reached over time (for example by means of determining the optical density at 600 nm or by means of determining the solid weight per unit of culture volume). The skilled person will understand that it is possible to monitor the culture to determine the percentage of lipids that accumulate in the biomass over time (for example by means of determining the amount of lipids per unit of mass in the culture using any of the aforementioned methods).

Once the desired amount of biomass and/or the desired intracellular amount of triglycerides has been reached, a second step of the first method of the invention comprises separating the microbial biomass from the culture medium. The cells are harvested by means of any of the methods normally used for this purpose, such as centrifugation, filtration, decantation, flotation or sedimentation, additionally aided by flocculation or evaporation to remove all or part of the water or medium from the aqueous fraction of the culture medium.

In a particular embodiment, the second step of the first method of the invention is performed by means of a method selected from the group consisting of filtration, microfiltration, centrifugation, pressure, decanting and combinations thereof.

In a particular embodiment, the method of the invention further comprises drying the microbial biomass obtained in the second step.

### Microbial biomass

In another aspect, the present invention also relates to the microbial biomass rich in triglycerides obtainable according to the first method of the invention, hereinafter "microbial biomass of the invention". The term "microbial biomass" has been described above and applies to the present aspect.

The microbial biomass generated according to the first method of the invention comprises not only the microorganisms but also all those components of the culture generated by the microorganisms or which have been incorporated in the microorganisms from the culture during growth and proliferation, such as nucleic acids, proteins, polysaccharides or lipids. The microbial biomass according to the invention comprises microorganisms of the *Rhodosporidium toruloides* CECT 13085 strain or of a mutant strain thereof, which maintains the capacity to accumulate lipids up to at least 50% of the dry weight, the capacity to grow in the presence of biomass hydrolysates without detoxification and the capacity to metabolize xylose. In a particular embodiment, the biomass rich in triglycerides contains an amount of the microorganism of the invention of at least 70%, at least 75%, at least 80%, at least 85%, at least 90%, at least 95% or greater with respect to the rest of the microorganisms present in the culture.

In a particular embodiment, the biomass rich in triglycerides has a triglyceride content that is at least 50% of the dry weight, at least 60% of the dry weight, at least 70% of the dry weight, or at least 80% of the dry weight.

### Method for extracting lipids from the microbial biomass

In another aspect, the present invention relates to a method for extracting lipids from the microbial biomass of the invention, hereinafter "second method of the invention".

The microorganisms that accumulate lipids and form part of the microbial biomass according to the present invention can be lysed to produce a lysate which is used as starting material for lipid extraction. The lysis step can be performed using any method known by a skilled person, such as, for example, heat lysis, lysis in basic medium, lysis in acid medium, enzymatic lysis using enzymes such as proteases or enzymes that degrade polysaccharides (amylases), lysis by ultrasonic means, mechanical lysis, osmotic shock lysis. These methods can be performed separately or in a combined manner, and in the event of a combined use, they can be performed simultaneously or sequentially. The degree of cell disruption can be determined by means of microscopic analysis.

The lysis step preferably requires disruption of at least around 70% of the cells, at least around 80% of the cells, at least around 90% of the cells, or preferably, at least around 100% of the cells.

Suitable methods for separating lipids from cell lysates include any mechanical-chemical extraction method, and among such methods, any solid-liquid extraction method. Suitable methods include extraction in the presence of organic solvents, which allows the expression of lipids and lipid derivatives such as fatty acid aldehydes and alcohols (Frenz et al. 1989, Enzyme Microb. Technol., 11:717), liquefaction (Sawayama et al. 1999, Biomass and Bioenergy 17:33-39 and Inoue et al. 1993, Biomass Bioenergy 6(4):269-274), liquefaction in oil (Minowa et al. 1995, Fuel 74(12):1735-1738), and extraction with supercritical CO₂.

The term "microbial biomass" has been described in detail in the context of the first method of the invention, and the definition and details thereof likewise apply to the second method of the invention.

The methods for extracting and determining the amount of lipids used in relation to the microorganism of the invention can likewise be applied in relation to the second method of the invention. Therefore, in a particular embodiment the mechanical extraction method is performed using a screw press, a French press or a ball mill.

In addition, lipids can also be extracted from the microbial biomass by using the differences in solubility thereof in a specific solvent. In the favorable case of a mixture of solids in which one of the compounds is soluble in a specific solvent (usually an organic solvent) while the other compounds are insoluble, extraction consisting of adding this solvent to the mixture contained in a beaker, flask or evaporating dish can be performed in cold or hot conditions, stirring or milling with the aid of a glass rod and separating the solution containing the extracted product and the insoluble fraction by filtration. Solid-liquid extraction is usually much more efficient when it is done continuously with the hot extraction solvent in a closed system, using a methodology similar to that explained above based on steeping the solid mixture to be extracted with organic solvent prior to steaming in a flask and condensing in a refrigerant. Transferring the organic solvent with part of the extracted product to the initial flask allows said organic solvent to be steamed again, repeating a new extraction cycle, whereas the non-volatile extracted product is concentrated in the flask.

Therefore, in another particular embodiment the solid-liquid extraction method is performed using a water-immiscible organic solvent. As it is used herein, the term "organic solvent" refers to a substance that dissolves a solute the molecules of which contain carbon atoms. As it is used herein, the term "water-immiscible organic solvent" refers to an organic solvent with little or no capacity for mixing with water. Non-limiting examples of water-immiscible organic solvents include n-hexane, acetone, petroleum ether and ethyl ether. Therefore, in a preferred embodiment said water-immiscible organic solvent is selected from the group consisting of n-hexane, acetone, petroleum ether, ethyl ether and combinations thereof. In an even more preferred embodiment, said water-immiscible organic solvent is n-hexane.

Solid-liquid extraction is usually much more efficient when it is done continuously with the hot extraction solvent in a closed system, using a methodology similar to that discussed for continuous liquid-liquid extraction, based on steeping the solid mixture to be extracted contained in a cellulose bag or cartridge placed in the extraction chamber with an organic solvent, previously steamed in a flask and condensed in a condenser. Transferring the organic solvent with part of the extracted product to the initial flask allows said organic solvent to be steamed again, repeating a new extraction cycle, whereas the non-volatile extracted product is concentrated in the flask.

### Method for obtaining products of industrial interest the biomass rich in lipids according to the invention

The lipids obtained from the microbial biomass according to the present invention can be chemically processed to give rise to products of interest in the industry. Examples of chemical modification methods that can be applied to the lipids according to the invention include lipid hydrolysis, lipid hydroprocessing and lipid esterification. Other chemical modifications include, without limitation, epoxidation, oxidation, hydrolysis, sulfation, sulfonation, ethoxylation, propoxylation, amidation and saponification. Modification of the lipids according to the present invention allows generating products that can be additionally modified to give rise to compounds of interest, such as soaps, fatty acids, fatty acid esters, fatty alcohols, fatty nitrogen compounds, fatty acid methyl esters and glycerol. Examples of oleochemical derivatives include, but are not limited to, fatty nitriles, esters, dimer acids, quaternary compounds, surface-active agents, fatty alkanolamides, fatty alcohol sulfates, resins, emulsifiers, fatty alcohols, olefins, drilling mud, polyols, polyurethanes, polyacrylates, rubber, candles, cosmetics, soaps, metal soaps, alpha-sulfonated methyl esters, fatty alcohol sulfates, fatty alcohol ethoxylates, fatty alcohol ether sulfates, imidazolines, surface-active agents, detergents, esters, quaternary compounds, ozonolysis products, fatty amines, fatty alkanolamides, ethoxy sulfates, monoglycerides, diglycerides, triglycerides (including medium-chain triglycerides), lubricants, hydraulic fluids, fats, dielectric fluids, mold release agents, metalworking fluids, heat transfer fluids, other functional fluids, industrial chemical products (for example cleaning products, auxiliary textile treatment products, plasticizers, stabilizers, additives), surface coatings, paints and lacquers, electric cable insulation, and higher alkanes.

### Method for obtaining paraffins

In another aspect, the present invention relates to a method for obtaining paraffins from the lipids obtained in the second method of the invention, hereinafter "third method of the invention", comprising
i) obtaining a lipid-enriched preparation from the microbial biomass of the invention
ii) refining the lipids obtained in step i), and
iii) converting the mixture of refined lipids obtained in step ii) into paraffins.

As it is used herein, the term "paraffin" refers to a group of alkane hydrocarbons of general formula CₙH₂ₙ₊₂, where n is the number of carbon atoms. The simple paraffin molecule comes from methane, CH₄, a gas at room temperature; in contrast, the heaviest members of the series, such as octane C₈H₁₈, are presented as liquids. Solid forms of paraffin, called paraffin wax, come from the heaviest C₂₀ to C₄₀ molecules. Non-limiting examples of paraffins include kerosene, diesel, biofuel, paraffin wax, nujol, adepsine oil, alboline, glymol, medicinal paraffin, saxol and USP mineral oil.

As it is used herein, the term "biofuel" refers to a fuel derived from biomass, such as animal, plant or microbial waste. Biofuels include, but are not limited to, biodiesel, biokerosene, biohydrogen, biogas, biomass derived from dimethylfuran (DMF) and the like. The term "biofuels" is also used to refer to fuel mixtures comprising combustible biomass derivatives, such as alcohol/gasoline mixtures (i.e., gasohols).

In a preferred embodiment of the third method of the invention, the paraffin is renewable diesel. In another preferred embodiment, the paraffin is biokerosene.

A first step of the third method of the invention comprises obtaining a lipid-enriched preparation according to the method of the second aspect of the invention.

A second step of the third method of the invention comprises refining the lipids obtained from the first step of said method.

As it is used herein, the term "refining" refers to the process of purifying a chemical substance many times obtained from a natural resource. Many methods are known in the state of the art for refining substances. For example, liquid refining is often achieved by distillation or fractionation. A gas can also be refined this way by cooling it or compressing it to liquefaction. Gases and liquids can also be refined by extraction with a solvent that dissolves either the substance of interest or the impurities.

Therefore, in a particular embodiment the lipids obtained in the second method of the invention are refined by means of at least one washing with NaOH at a concentration between 5% and 15%. The refining process comprises at least one washing with NaOH, at least two washings with NaOH, at least three washings with NaOH, at least four washings with NaOH, at least five washings with NaOH, at least ten washings with NaOH, or more. In a preferred embodiment, the NaOH concentration is between 8% and 12%. In a more preferred embodiment, the NaOH concentration is 10%.

A third step of the third method of the invention comprises converting the mixture of refined lipids obtained in step ii) into paraffins.

In a particular embodiment, step ii) of the third method of the invention comprises a method selected from the group consisting of hydrotreating or hydroprocessing

The person skilled in the art will understand that there are many methods in the art for converting lipids into paraffins including, without limitation, hydrotreating or hydroprocessing methods (EP1682466, EP1795576, EP1681337, EP1640437).

As it is used herein, the term "hydrotreating" or "hydroprocessing" refers to usually catalytic hydrogenation reactions that are widely used on petroleum fractions such as naphtha, kerosene and diesel, as well as lipid fractions, under high pressure and temperature. In the particular case of the present invention, hydroprocessing is performed on the mixture of lipids obtained in the second method of the invention.

Hydroprocessing is necessary to remove contaminants such as nitrogen, sulfur metals, and heavy metals from fuel oils. Oxygenated hydrocarbons thereby substitute their oxygen atoms with hydrogen atoms, and the exiting oxygen atoms combine with hydrogen molecules, forming water. Nitrogenous hydrocarbons substitute their nitrogen atoms with hydrogen atoms, and the exiting nitrogen atoms combine with hydrogen molecules, forming ammonia. Finally, hydrocarbons containing sulfur substitute their sulfur atoms with hydrogen atoms, and the exiting sulfur atoms combine with hydrogen molecules, forming hydrogen sulfide. Once the hydroprocessing method has been performed, paraffins are separated from the rest of the substances and subjected to other treatments until achieving the desired characteristics.

As it is used herein, the term "catalysis" refers to the increase in the rate of a chemical reaction due to the participation of a substance referred to as a catalyst. Unlike other reagents in the chemical reaction, a catalyst is not consumed. A catalyst can participate in multiple chemical transformations. The effect of a catalyst can vary due to the presence of other substances known as inhibitors or poisons (which reduce catalytic activity) or promoters (which increase activity). In the context of the present invention, the main catalysts useful in hydroprocessing are based on molybdenum disulfide (MoS₂) together with smaller amounts of other metals. Most metals catalyze hydroprocessing, but those metals in the middle of the series of transition metals are more active. Ruthenium disulfide seems to be the most active catalyst, but binary combinations of cobalt and molybdenum are also extremely active. Besides base cobalt modified with MoS₂, catalyst, nickel and tungsten are also used. For example, Ni-W catalysts are the most effective catalysts for hydrodenitrogenation.

Another hydrotreating method comprises contacting the refined lipids with water, applying high temperature and pressure, and separating the organic phase from water. Another hydroprocessing method comprises hydrogenating the mixture of refined lipids obtained in the step, and furthermore deoxygenating said mixture of refined lipids.

In a preferred embodiment, the hydrotreating method comprises
- contacting the mixture of refined lipids obtained in step ii) with water,
- applying high temperature and pressure, and
- separating the organic phase from water.

In this embodiment, hydrotreatment is performed in liquid phase, at a high temperature of 100 to 400°C, preferably 250 to 350°C. The reaction can be carried out at atmospheric pressure. However, to keep the reagents in the liquid phase, it is preferable to use a pressure that is greater than steam saturation pressure, and therefore reaction pressure intervals range from atmospheric pressure to 20 MPa, preferably 0.1 to 5 MPa.

Once the reaction has ended, the organic phase is separated from water, obtaining a distillate as a product with the composition of a renewable diesel.

In another preferred embodiment, the hydroprocessing method comprises
- hydrogenating the mixture of refined lipids obtained in step ii), and
- deoxygenating said mixture of refined lipids.

In a more preferred embodiment, the hydroprocessing method is performed at high temperature and pressure.

As it is used herein, the term "deoxygenation" refers to a chemical reaction involving the removal of molecular oxygen (O₂) from a reaction mixture or solvent, or the removal of the oxygen atoms from a molecule. Non-limiting examples of deoxygenation reactions include substituting a hydroxyl group with hydrogen in Barton-McCombie deoxygenation or in Markó-Lam deoxygenation, and substituting an oxo group with two hydrogen atoms in Wolff-Kishner reduction.

For example, lipids and optionally a solvent or a mixture of solvents are contacted with a heterogeneous decarboxylation catalyst selected from catalysts containing one or more group VIII and/or VIA metals from the periodic chart. The catalysts are preferably of alumina, silica and/or carbon-supported Pd, Pt, Ni, NiMo or CoMo catalysts. Hydrogen can optionally be used. Decarboxylation reaction conditions vary with the raw material used. The reaction is performed in liquid phase, at a high temperature from 100 to 400°C, preferably 250 to 350°C. The reaction can be performed at atmospheric pressure. However, to keep the reagents in the liquid phase, it is preferable to use a pressure that is greater than steam saturation pressure, and therefore reaction pressure intervals range from atmospheric pressure to 150 MPa, preferably 0.1 to 5 MPa.

In a preferred embodiment, hydrogenation and deoxygenation of said mixture of refined lipids is performed in the same step. In another preferred embodiment, hydrogenation and deoxygenation of said mixture of refined lipids is performed in consecutive steps.

The product that is obtained once the reaction has ended is a renewable diesel.

In another particular embodiment, the third method of the invention additionally comprises a catalytic cracking process in conditions suitable for converting the paraffins obtained in step iii) in biokerosene.

As it is used herein, the term "catalytic cracking" refers to breaking up a long-chain alkane into other more useful short-chain alkanes and alkenes, i.e., the process of breaking up long-chain hydrocarbons into short-chain hydrocarbons. It is a process of thermal decomposition in the presence of a catalyst of the components of the paraffins obtained by means of hydrotreating methods, for the purpose of cracking long-chain hydrocarbons the boiling point of which is equal to or greater than 315°C, and converting them into short-chain hydrocarbons the boiling point of which is below 221°C. Said catalysts are in granular or microspherical form. Catalysts are usually made up of a silicon oxide (SiO₂) and alumina (Al₂O₃). The most commonly used mineral for this purpose is faujasite.

In a preferred embodiment, said catalytic cracking uses a solid catalyst.

As it is used herein, the term "solid catalyst" refers to a chemical, solid, simple or composite substance that modifies the rate of a chemical reaction, being involved in it but without being part of the products resulting from same. Most solid catalysts are metals or oxides, sulfides and haloids of metallic and semimetallic elements, such as boron, aluminum, and silicon elements. Solid catalysts can be prepared by means of precipitation-deposition consisting of depositing a hydroxide by means of precipitation of a soluble salt of the metal on the support. In this case, precipitation is primarily performed by modifying the solution pH. The most widely used method due to its simplicity is impregnation, which consists of adding the support to a solution with the desired active phase content, and removing the solvent by evaporation. In a more preferred embodiment, said solid catalyst is selected from the group consisting of catalysts consisting of bifunctional metallic hydrogenation-dehydrogenation systems (for example Co-Mo or Pd-Pt) and acidic cracking components (for example Al₂O₃, SiO₂, and also in the form of zeolites) in the presence of hydrogen.

### Method for obtaining biodiese)

In another aspect, the present invention relates to a method for obtaining biodiesel from the lipids obtained in the second method of the invention, hereinafter "fourth method of the invention", comprising
i) obtaining a lipid-enriched preparation from the microbial biomass of the invention,
ii) refining the lipids obtained in step i), and
iii) converting the mixture of refined lipids obtained in step ii) into biodiesel.

As it is used herein, the term "biodiesel" refers to a chemical composition essentially made up of long-chain fatty acid mono-alkyl esters. The esters that are part of biodiesel are methyl, ethyl or propyl esters, and the fatty acids are those that come from the lipid composition according to the present invention. In preferred embodiments, the biodiesel according to the present invention comprises one or several of the following fatty acid alkyl esters: fatty acid methyl esters (FAME), fatty acid ethyl esters (FAEE), fatty acid butyl esters (FABE). In particular embodiments, the biodiesel can contain one or several fatty acids selected from myristate, palmitate, stearate, oleate, linolenate, arachidate and behenate. In a preferred embodiment of the invention, the biodiesel is a fuel which is made up entirely of esters of a biological origin that do not contain diesel coming from petroleum and comprise long-chain fatty acid mono-alkyl esters. This type of biodiesel is known as B100 and this indicates that 100% of the fuel is biodiesel

The person skilled in the art will understand that there are many methods in the art for converting lipids into biodiesel, including, without limitation, transesterification methods (EP1682466, EP1795576, EP1681337, EP1640437).

As it is used herein, the term "esterification" or "transesterification" refers to the reaction taking place between a fatty acid and an alcohol. The product of said reaction is a fatty acid ester. Transesterification can be base-, acid- or enzyme-catalyzed transesterification. In one embodiment, the biodiesel is obtained from the lipid preparation according to the invention by means of transesterification of the free fatty acids that are part of the lipid preparation.

The acid-catalyzed transesterification process is performed in the presence of Brönsted acids, preferably sulfonic or sulfuric acid. These catalysts generate very high alkyl ester production but the reactions are slow compared to alkaline catalysts. This type of transesterification is typically used with those lipids having a high free fatty acid content.

The base-catalyzed transesterification process is performed with methanol through alkaline. The catalyst (for example NaOH, KOH, NaHCO₃, KHCO₃) is dissolved in alcohol and after adding it to the oil, the mixture is stirred at a certain temperature and pressure, which could be adjusted according to experimental conditions. This reaction gives rise to fatty acid esters and crude glycerine as final reaction products.

The lipase-catalyzed enzymatic transesterification process is performed in the presence of lipase enzymes or lipase-producing microorganisms, such as, for example, those belonging to the genera *Candida sp, Chromobacteri sp, Cryptococcus sp, Mucor sp, Pseudomonas sp, Rhizomucor sp, Rhizopus sp, Thermomyces sp,* etc.

In an even more particular embodiment, biodiesel is obtained by means of base-catalyzed transesterification as shown in Example 6 of the present invention. As the skilled person will understand, the base catalyst concentration, the amount of substrate, the temperature and the reaction time may be adjusted. The obtained reaction products are methyl esters of the corresponding fatty acids forming the biodiesel and glycerine. Methanol used as a diluent of the catalyst can be removed by means of different physicochemical methods known in the state of the art, such as heat treatment, distillation, etc. Due to the different density of the reaction products, the light phase, which will contain glycerine and other compounds, can be separated from the heavy phase, formed by fatty acid methyl esters, by means of any known technique that allows separating liquids having different densities, such as, for example, centrifugation, sedimentation, filtration, crystallization, etc.

The obtained methyl esters can optionally be purified to remove impurities (small amounts of methanol, glycerine, catalyst, soaps, cell debris and compounds having a high boiling point). Methods for purifying methyl esters are well known in the state of the art and include, without being limited to, methods of chromatographic purification, crystallization, vacuum distillation, or washing with diluted acid solutions. In an even more particular embodiment, the step of purifying the obtained methyl esters is performed by means of washing with a diluted hydrochloric acid solution. This washing allows removing insoluble impurities that are found with the ester and is able to prevent the formation of emulsions. Washing is typically performed at the same temperature as that used in the transesterification reaction. After washing, the aqueous phase and organic phase of the mixture are separated. As the skilled person will understand, any technique that allows separating the organic phase and the aqueous phase may be used in the context of the present invention. Though not limited to such methods, both phases can be separated by means of extracting the organic phase, decanting or evaporating the aqueous phase. Finally, the organic phase, which is what will contain the methyl esters, still entrains a considerable portion of water that must be removed. The step of drying is typically done in high pressure and temperature conditions (temperatures around 100°C and usually applying a vacuum).

### Method for obtaining biolubricants

In another aspect, the present invention relates to a method for obtaining biolubricants from the lipids obtained in the second method of the invention, hereinafter "fifth method of the invention", comprising:
i) obtaining a lipid-enriched preparation from the microbial biomass of the invention,
ii) refining the lipids obtained in step i), and
iii) converting the mixture of refined lipids obtained in step ii) into biolubricants.

As it is used herein, the term "biolubricant" refers to a lubricant derived from the biomass, such as animal, plant or microbial waste that is not toxic for animal life or aquatic life and can be degraded by means of the action of microorganisms in a relatively short period of time.

A first step of the fifth method of the invention comprises obtaining a lipid-enriched preparation according to the method of the second aspect of the invention.

A second step of the fifth method of the invention comprises refining the lipids obtained from the second method of the invention. The term "refining" has been defined for the third method of the invention as well as for particular embodiments thereof and likewise applies to the fourth method of the invention.

A third step of the fifth method of the invention comprises converting the mixture of refined lipids obtained in step ii) into biolubricants. The person skilled in the art will understand that there are many methods in the art for converting lipids into biolubricants, including, without limitation, the methods shown in documents (Petran et al. 2008. Goriva i Maziva, 47: 463-478, US8124572 and US5713965). Said methods are based on transesterification methods and subsequent chemical modification of the obtained free fatty acids. The term "transesterification" has been defined above in the context of the third method of the invention and similarly applies to the fourth method of the invention. In a preferred embodiment, after the step of transesterification the fatty acids can be separated according to the number of double bonds they contain in their structure by means of any method known in the state of the art (Biochem. J., vol., 62, p. 222, 1956). Monounsaturated fatty acids are generally selected for use as lubricants. Said fatty acids can be modified by means of esterification, giving rise to unsaturated fatty acid esters, and epoxidation. The species formed after this step (epoxy-fatty acid esters) can finally be chemically modified by means of esterification with different groups, such as carboxylic acids, halic acid, acyl anhydrides, etc.

### Method for obtaining biosurfactants

In another aspect, the present invention relates to a method for obtaining biosurfactants from the lipids obtained in the second method of the invention, hereinafter "sixth method of the invention", comprising:
i) obtaining a lipid-enriched preparation from the microbial biomass of the invention,
ii) refining the lipids obtained in step i), and
iii) converting the mixture of refined lipids obtained in step ii) into biosurfactants.

As it is used herein, the term "biosurfactant" refers to an amphipathic compound which has the capacity to interact with hydrophobic and hydrophilic compounds at the same time, which is derived from the biomass, such as animal, plant or microbial waste. Said term includes, without limitation, rhamnolipids, trehalolipids, sophorolipids, cellobiolipids, polyol lipids, diglycosyl diglycerides, lipopolysaccharides, arthrofactin, surfactin, viscosin, phospholipids and sulfonylipids.

A first step of the sixth method of the invention comprises obtaining a lipid-enriched preparation according to the method of the second aspect of the invention.

A second step of the sixth method of the invention comprises refining the lipids obtained from the second method of the invention. The term refining has been defined for the third method of the invention as well as particular embodiments thereof, and likewise applies to the fourth method of the invention.

A third step of the sixth method of the invention comprises converting the mixture of refined lipids obtained in step ii) into biosurfactants. The person skilled in the art will understand that there are many methods in the art for converting lipids into biosurfactants including, without limitation, methods of... (see, for example, O'Lenick. 1999. Surfactants: Chemistry & Properties; Levinson. 2008. Surfactant production, pp.: 1-37. CRC Press).

In a preferred embodiment, the surfactant is a fatty acid, which can be obtained from the lipid preparation according to the invention by means of saponification of the triglycerides that are part of the lipid preparation.

In another preferred embodiment, the surfactant is a monoglyceride, which can be obtained from the lipid preparation according to the invention by means of glycerolysis of the triglycerides that are part of the lipid preparation.

In another preferred embodiment, the surfactant is an alkyl polyglucoside (APG), which can be obtained from the lipid preparation according to the invention by means of the steps of (i) saponification of the triglycerides according to the invention to give rise to fatty acids, (ii) hydrogenation of the fatty acids to give rise to alcohols, and (iii) acetalization (or transacetalization) with glucose of fatty acid alcohols.

In another preferred embodiment, the surfactant is a fatty acid ester, which can be obtained from the lipid preparation according to the invention by means of the steps of (i) saponification of the triglycerides that are part of the lipid preparation according to the invention to give rise to fatty acids, and (ii) condensation with ethanolamine to give rise to ethoxylates.

### Uses of the invention

In another aspect, the present invention relates to the use of the microorganism of the invention, hereinafter "first use of the invention", for obtaining a microbial biomass rich in triglycerides according to the first method of the invention.

The terms "microorganism" and "microbial biomass rich in triglycerides" and their particularities have been described in the context of the microorganism and of the first method of the invention and apply to the first use of the invention. Preferred particular embodiments of the microorganism and of the first method of the invention also apply in a similar manner.

In another aspect, the present invention relates to the use of the microorganism of the invention, hereinafter "second use of the invention", for extracting lipids from the microbial biomass according to the second method of the invention.

The terms "microorganism" and "microbial biomass" and their particularities have been described in the context of the microorganism and of the first method of the invention, and apply to the second use of the invention. Preferred particular embodiments of the microorganism and of the second method of the invention also apply in a similar manner.

In another aspect, the present invention relates to the use of the microorganism of the invention, hereinafter "third use of the invention", for obtaining paraffins according to the third method of the invention.

In another aspect, the present invention relates to the use of the microorganism of the invention, hereinafter "fourth use of the invention", for obtaining biodiesel according to the fourth method of the invention.

In another aspect, the present invention relates to the use of the microorganism of the invention, hereinafter "fifth use of the invention", for obtaining biolubricants according to the fifth method of the invention.

In another aspect, the present invention relates to the use of the microorganism of the invention, hereinafter "sixth use of the invention", for obtaining biosurfactants according to the sixth method of the invention.

The terms "microorganism", "paraffin", "biodiesel", "biolubricant" and "biosurfactant" and their particularities have been described in the context of the microorganism and of the third, fourth, fifth and sixth methods of the invention, and apply to the third, fourth, fifth and sixth uses of the invention. Preferred particular embodiments of the microorganism and of the third, fourth, fifth and sixth methods of the invention also apply in a similar manner.

The invention is described below in detail by means of the following examples, which must be interpreted as being merely illustrative and not limiting of the scope of the invention.

### Examples

### Example 1: Obtaining Rhodosporidium toruloides CECT 13085

This strain was obtained from the superproducer strain R. *toruloides* 0041-12 by applying standard enriching, mutation and selection methods (Adrio and Demain. 2006. FEMS Microbiol. Rev. 30: 187-214). The microorganism has been deposited in the Spanish Type Culture Collection as *Rhodosporidium toruloides* CECT 13085.

The capacity to accumulate fats was analyzed by means of acid hydrolysis and intracellular fat extraction with hexane following the method described in Kolar et *al.* (Kolar et al. 1993. J. Anal. Chem., 347: 393-395).

By means of this gravimetric measurement, it was confirmed that the strain was capable of accumulating over 50% of its dry weight in the form of lipids. This strain was identified by means of PCR amplification of the D1/D2 region of 28S gene and the ribosomal DNA intergenic region (ITS-5.8S), followed by sequencing of both and comparison with databases (NCBI/Blast).

The microorganism has been deposited in the Spanish Type Culture Collection as *Rhodosporidium toruloides* CECT 13085.

### Example 2: Growth in biomass hydrolysates

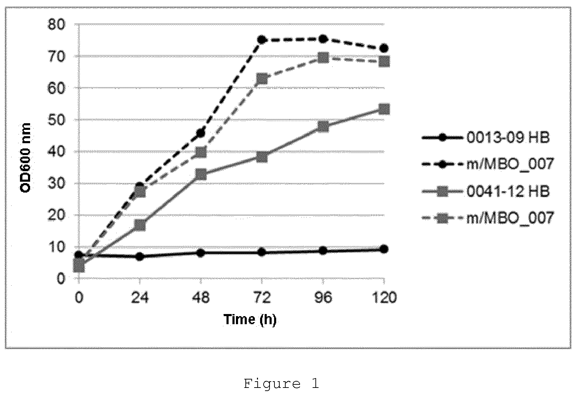

The biomass hydrolysates obtained by treating wheat straw by means of steam explosion in acid medium were hydrolyzed using different solid concentrations (4-20% w/w). The resulting solutions containing 20-100 g of sugars/liter were used to prepare culture media. *R. toruloides* 0013-09 was grown in flasks containing m/MBO_008 medium (composition: 9.6 g/l corn steep liquid, 20 g/l sugars, pH 6) at 30°C, 250 rpm for 24 hours. These cultures were used to inoculate 50 ml of the m/MBO_008_1 medium (composition: 9.6 g/l corn steep liquid, 70 g/l sugars coming from wheat straw, pH 6). The cultures were kept at 30°C, 250 rpm for 5 days. As shown in Figure 1, the *R. toruloides* 0013-09 strain was not capable of growing due to the high inhibitory compound concentrations present in this culture medium.

For the purpose of obtaining a strain capable of being resistant to and growing in the presence of these high toxic compound concentrations, this strain was developed by adaptation. To that end, successive rounds of culture were done in 50 ml flasks containing m/MBO_008_1 medium and by transferring 10% of the volume of the culture to another flask containing fresh medium every 24 hours. A total of 25 rounds where thereby completed. A strain (0041-12) that showed good growth in the presence of these high inhibitor concentrations, as can be seen in Figure 1, was finally selected.

### Example 3: Improvement of xylose metabolism

*R. toruloides* 0041-12 cells grown in YPD medium (composition: 10 g/l yeast extract, 20 g/l glucose, 20 g/l peptone) for 16 hours at 30°C and 250 rpm were harvested by centrifugation and resuspended in 50 mM potassium phosphate buffer pH 8. 10 ml aliquots of said suspension were treated with N-methyl-N-nitro-N-nitrosoguanidine (NTG, 0.15 mg/ml) and incubated under stirring at 30°C for 45 minutes. Culture viability at this end point was less than 1%.

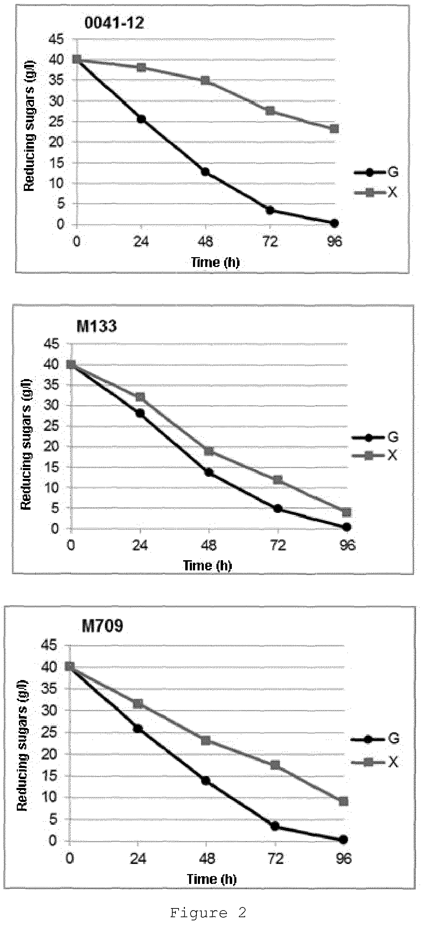

Surviving cells were seeded on plates containing m/MBO_002 medium (composition: 1 g/l NH₄NO₃, 0.4 g/l CaCl₂•H₂O, 0.76 g/l KH₂PO₄, 0.4 g/l MgSO₄-7H₂O, 20 g/l xylose, 20 g/l agar, pH 6) and incubated at 30°C for 5 days. A total of 785 colonies showed faster growth and were selected as possible candidates. These colonies were then subjected to a second round of selection in 96-well plates containing 0.4 ml of m/MB02_002 medium (composition: 0.96 g/l corn steep liquid, 40 g/l glucose or xylose, pH 6). 87 strains that showed similar or even better growth when xylose was used as the only carbon source were selected. These colonies were subsequently cultured in 10 ml of m/MB02_004 medium (composition: 9.6 g/l corn steep liquid, 40 g/l glucose or xylose, pH 6). In this third round, 21 strains that showed similar growth in both sugars were selected. Among such strains, M709 and M133 showed the best growth and specific growth rates in xylose (µxyl) with respect to the parental strain (Table 1 and Figure 2). Of these two strains, M133 was capable of consuming all the xylose present in the culture media more rapidly (Figure 2).

The M133 strain has been deposited in the Spanish Type Culture Collection (CECT) with the accession number 13085.

**Table 1. Specific growth rate and duplication time in glucose and xylose**

| | Carbon source | µₙₑₜ (h⁻¹) | Td (h) | R² |
|---|---|---|---|---|
| M133 | Glucose Xylose | 0.2956 | 2.3 | 0.9915 |
| | | 0.1339 | 5.2 | 0.9985 |
| M709 | Glucose Xylose | 0.3173 | 2.2 | 0.9978 |
| | | 0.1548 | 4.5 | 0.9944 |
| 0041-12 | Glucose Xylose | 0.3263 | 2.1 | 0.998 |
| | | 0.071 | 9.8 | 0.9723 |

| | | | | |
|---|---|---|---|---|
| µₙₑₜ (h⁻¹) : specific growth rate (hours ⁻¹) Td (h): duplication time (hours) R²: coefficient of correlation | | | | |

### Example 4: Production of oil different types of biomass hydrolysates

Cultures of *R. toruloides* CECT 13085 grown in m/MBO_008 medium (composition: 9.6 g/l corn steep liquid, hydrolysate up to a sugar concentration of 20 g/l, pH 6) were used to inoculate 500 ml flasks containing 100 ml of m/MBO_008_1 1 medium (composition: 9.6 g/l corn steep liquid, 70 g sugars/l of wheat straw hydrolysate, pH 6), of m/MBO_008_2 medium (composition: 9.6 g/l corn steep liquid, 70 g sugars/l of sugarcane bagasse hydrolysate, pH 6), or of m/MBO_008_3 medium (composition: 9.6 g/l corn steep liquid, 70 g sugars/l of hydrolysate of oil palm empty fruit bunches, pH 6). The cultures were kept at 30°C, 250 rpm for 7 days. Once the cultures finished, the cells were harvested by centrifugation and the biomass of each flask was oven-dried at 65°C for 48 hours under constant stirring, obtaining 1.70 g, 1.68 g and 1.72 g, respectively. Using the biomass of each flask, the oil content was gravimetrically analyzed by means of extraction with n-hexane. 1 gram, 1.1 grams and 1 gram of oil were finally obtained, which correspond to an intracellular oil content of 61%, 65% and 58%, respectively.

### Example 5: Production of oil wheat straw hydrolysate and crude glycerine

Cultures of *R. toruloides* CECT 13085 grown in m/MBO_008 medium (composition: 9.6 g/l corn steep liquid, hydrolysate up to a sugar concentration of 20 g/l, pH 6) were used to inoculate 500 ml flasks containing 100 ml of m/MBO_008_4 medium (composition: 9.6 g/l corn steep liquid, 52 g/l sugars of wheat straw hydrolysate, 18 g/l crude glycerine, pH 6). The culture was kept at 30°C, 250 rpm for 7 days. Once the culture finished, the cells were harvested by centrifugation and the biomass was oven-dried at 65°C for 48 hours under constant stirring, obtaining 1.8 g. Using this sample, the oil content was gravimetrically analyzed by means of extraction with n-hexane. 1.2 g of oil were obtained, which corresponds to an intracellular content of 64%.

### Example 6: Production of biodiesel

An extracted and refined oil sample (0.5 kg) taken from the *Rhodosporidium toruloides* CECT 13085 strain was used to perform the transesterification reaction. The reaction was performed in three steps, each lasting for 2 hours and at a temperature of 55°C. NaOH (1% w/v) and methanol (10% v/v) were added in each step. Once the reaction ended, stirring was stopped and the mixture was separated by means of centrifugation. Two phases were obtained: a light phase containing excess methanol and methyl esters, and a heavy phase formed by glycerine, methanol residues, catalyst and salts.

The light phase was purified by means of four steps of washing at 55°C. HCl (2%) was used in the first step, and distilled water was used in the remaining three steps. At the end of each washing, the mixture was left to decant until good separation of the organic phase and aqueous phase was achieved. The aqueous phase was removed, and the methyl esters were subjected to a step of drying to remove methanol residues and water by means of evaporation under a vacuum and at 115°C. The final amount obtained was 0.49 kg of methyl esters, which represents a 98% yield. Analysis of the obtained biodiesel complied with all the parameters required by the EN14214 standard.

### Deposits of Biological Material

The *Rhodosporidium toruloides* strain characterized by its capacity to accumulate lipids up to at least 50% of the dry weight, by its capacity to be resistant to biomass hydrolysates without detoxification, and by its capacity to metabolize xylose has been deposited in the Spanish Type Culture Collection under the conditions provided in the Budapest Treaty. The material was deposited on May 7, 2013, and the number assigned to said deposit was CECT 13085.

## Claims

1. A microorganism of the *Rhodosporidium toruloides* CECT 13085 strain or of a mutant strain thereof, which maintains the capacity to accumulate lipids up to at least 50% of the dry weight, the capacity to grow in the presence of biomass hydrolysates without detoxification and the capacity to metabolize xylose.

2. A method for obtaining a microbial biomass rich in triglycerides, comprising
i) culturing a microorganism according to claim 1 in a culture medium comprising at least one carbon source and at least one nitrogen source in conditions suitable for growth of said microorganism, and
ii) separating the microbial biomass from the culture medium.

3. The method according to claim 2, wherein the carbon source is a lignocellulosic biomass hydrolysate.

4. The method according to claim 3, wherein the carbon source further comprises glycerol.

5. The method according to claim 3 or 4, wherein the hydrolysate is obtained from wheat straw, sugarcane bagasse, corn stover, oil palm empty fruit bunches, oil palm pruning, oil palm fiber, grapevine pruning, olive tree pruning and combinations thereof.

6. The method according to claim 2, wherein the carbon source is selected from the group consisting of glucose, glycerol, molasses, xylose, arabinose, mannose, fructose, acetate and combinations thereof.

7. The method according to claim 6, wherein the carbon source is glucose.

8. The method according to claim 6, wherein the carbon source is xylose.

9. The method according to any of claims 2 to 8, wherein the nitrogen source is selected from the group consisting of yeast extract, peptone, corn steep liquid, urea, sodium glutamate, different inorganic nitrogen sources, such as ammonium salts and combinations thereof.

10. The method according to any of claims 2 to 9, wherein the nitrogen source is an ammonium salt, preferably ammonium chloride.

11. The method according to any of claims 2 to 10, wherein the culture medium contains solid inhibitors selected from: acetic acid, formic acid, levulinic acid, coumaric acid, ferulic acid, succinic acid, 4-hydroxybenzaldehyde, vanillin, vanillic acid, syringaldehyde, 4-hydroxybenzoic acid, catechol, guaicol, syringic acid, furfural, 5-hydroxymethylfurfural and combinations thereof.

12. The method according to any of claims 2 to 11, wherein conditions suitable for growth of said microorganism of step (i) comprise
- temperature in a range between 18°C and 37°C,
- dissolved oxygen concentration of at least 20%, and/or
- constant stirring.

13. The method according to any of claims 2 to 12, wherein step ii) is performed by means of a method selected from the group consisting of filtration, microfiltration, centrifugation and combinations thereof.

14. The method according to any of claims 2 to 13, further comprising drying the microbial biomass of step ii).

15. A microbial biomass rich in triglycerides obtainable according to the method of any of claims 2 to 14.

16. A method for obtaining a lipid-enriched preparation comprising
i) obtaining a microbial biomass according to claim 15 and
ii) extracting the lipids from said microbial biomass.

17. The method according to claim 16, wherein the extraction of step (ii) is performed by means of mechanical methods or by means of a solid-liquid extraction method.

18. The method according to claim 17, wherein the mechanical extraction method is performed using a screw press, a French press or a ball mill.

19. The method according to claim 18, wherein the solid-liquid extraction method is performed using a water-immiscible organic solvent.

20. The method according to claim 19, wherein said water-immiscible organic solvent is selected from the group consisting of n-hexane, acetone, petroleum ether, ethyl ether and combinations thereof.

21. A method for obtaining paraffins comprising the steps of:
i) obtaining a lipid-enriched preparation from a microbial biomass according to claim 15,
ii) refining the lipids obtained in step ii), and
iii) converting the mixture of refined lipids obtained in step ii) into paraffins.

22. The method according to claim 21, wherein step ii) is performed by means of at least one washing with NaOH at a concentration between 5% and 15%.

23. The method according to any of claims 21 or 22, wherein step iii) comprises a hydrotreating or hydroprocessing method.

24. The method according to claim 23, wherein the hydrotreating method comprises
- contacting the mixture of refined lipids obtained in step ii) with water,
- applying high temperature and pressure, and
- separating the organic phase from water.

25. The method according to claim 23, wherein the hydroprocessing method comprises
- hydrogenating the mixture of refined lipids obtained in step ii), and
- deoxygenating said mixture of refined lipids.

26. The method according to claim 25, wherein the hydrogenation and deoxygenation of said mixture of refined lipids is performed in the same step or in consecutive steps.

27. The method according to any of claims 25 or 26, wherein the hydroprocessing method is performed at high temperature and pressure.

28. The method according to any of claims 24 to 27, additionally comprising a catalytic cracking process in conditions suitable for converting the paraffins obtained in step ii) into biokerosene.

29. The method according to claim 28, wherein said catalytic cracking uses a solid catalyst.

30. A method for obtaining biodiesel comprising the steps of
i) obtaining a lipid-enriched preparation from the microbial biomass according to claim 15,
ii) refining the lipids obtained in step i), and
iii) converting the mixture of refined lipids obtained in step ii) into biodiesel.

31. A method for obtaining biolubricants comprising the steps of
i) obtaining a lipid-enriched preparation from the microbial biomass according to claim 15,
ii) refining the lipids obtained in step i), and
iii) converting the mixture of refined lipids obtained in step ii) into biolubricants.

32. A method for obtaining biosurfactants comprising the steps of::
i) obtaining a lipid-enriched preparation from the microbial biomass according to claim 15,
ii) refining the lipids obtained in step i), and
iii) converting the mixture of refined lipids obtained in step ii) into biolubricants.

33. Use of the microorganism according to claim 1 or of the microbial biomass rich in triglycerides according to claim 15 for obtaining a lipid-enriched preparation, for obtaining paraffins, for obtaining biodiesel, for obtaining biolubricants or for obtaining biosurfactants.
